# EUROPEAN PATENT APPLICATION

(11) **EP 1 722 309 A1**
(43) Date of publication of application: **15.11.2006**
(21) Application number: 05010351.4
(22) Date of filing: 12.05.2005
(51) Int. Cl.: G06F 19/00

(54) **Method of normalizing gene expression data**

(71) Applicant: MAX-PLANCK-GESELLSCHAFT ZUR FÖRDERUNG DER WISSENSCHAFTEN E.V., 14195 Berlin (DE)
(72) Inventor: Jacobson, Marc, 13347 Berlin (DE); Nietfeld, Wilfried, 10829 Berlin (DE); Mollenkopf, Hans, 10825 Berlin (DE); Kaufmann, Stefan, 13465 Berlin-Frohnau (DE); Repsilber, Dirk, 23566 Lübeck (DE); Ziegler, Andreas, 23611 Sereetz (DE)
(74) Representative: Vossius & Partner

(57) **Abstract**

This invention relates to a method of normalizing gene expression data, wherein said gene expression data are obtained from a sample out of a plurality of samples, the sample reflecting one or more cell type(s), and the method comprising the steps of: (a) determining the expression level in said sample of one or more first genes the expression of which is specific for one or more chosen cell type(s) and is constant per cell across said plurality of samples (constant marker genes); and (b) normalizing the expression level of one or more second genes in said sample using the expression level(s) determined in step (a). Also provided is a computer program comprising program code means for performing the method of the invention.

SEQUENCE LISTING

<110>  Max-Planck-Gesellschaft zur Förderung der Wissenschaften e.V.

<120>  Method of normalizing gene expression data

<130>  K 2927 EP

<160>  4

<170>  PatentIn version 3.1

<210>  1
<211>  3982
<212>  DNA
<213>  Homo sapiens

<400>  1
   cacagctgct tgctgctcca gccttgccct cccagagctg ccggacgctc gcgggtctcg      60
   gaacgcatcc cgccgcgggg gcttcggccg tggcatgggc gccgcgggcc tgctcggggt     120
   tttcttggct ctcgtcgcac cgggggtcct cgggatttct tgtggctctc ctccgcctat     180
   cctaaatggc cggattagtt attattctac ccccattgct gttggtaccg tgataaggta     240
   cagttgttca ggtaccttcc gcctcattgg agaaaaaagt ctattatgca taactaaaga     300
   caaagtggat ggaacctggg ataaacctgc tcctaaatgt gaatatttca ataaatattc     360
   ttcttgccct gagcccatag taccaggagg atacaaaatt agaggctcta caccctacag     420
   acatggtgat tctgtgacat ttgcctgtaa aaccaacttc tccatgaacg gaaacaagtc     480
   tgtttggtgt caagcaaata atatgtgggg gccgacacga ctaccaacct gtgtaagtgt     540
   tttccctctc gagtgtccag cacttcctat gatccacaat ggacatcaca caagtgagaa     600
   tgttggctcc attgctccag gattgtctgt gacttacagc tgtgaatctg gttacttgct     660
   tgttggagaa aagatcatta actgtttgtc ttcgggaaaa tggagtgctg tcccccccac     720
   atgtgaagag gcacgctgta aatctctagg acgatttccc aatgggaagg taaaggagcc     780
   tccaattctc cgggttggtg taactgcaaa ctttttctgt gatgaagggt atcgactgca     840
   aggcccacct tctagtcggt gtgtaattgc tggacaggga gttgcttgga ccaaaatgcc     900
   agtatgtgaa gaaatttttt gcccatcacc tccccctatt ctcaatggaa gacatatagg     960
   caactcacta gcaaatgtct catatggaag catagtcact tacacttgtg acccggaccc    1020
   agaggaagga gtgaacttca tccttattgg agagagcact ctccgttgta cagttgatag 1080
   tcagaagact gggacctgga gtggccctgc cccacgctgt gaactttcta cttctgcggt 1140
   tcagtgtcca catccccaga tcctaagagg ccgaatggta tctgggcaga aagatcgata 1200
   tacctataac gacactgtga tatttgcttg catgtttggc ttcaccttga agggcagcaa 1260
   gcaaatccga tgcaatgccc aaggcacatg ggagccatct gcaccagtct gtgaaaagga 1320
   atgccaggcc cctcctaaca tcctcaatgg gcaaaaggaa gatagacaca tggtccgctt 1380
   tgaccctgga acatctataa aatatagctg taaccctggc tatgtgctgg tgggagaaga 1440
   atccatacag tgtacctctg agggggtgtg gacaccccct gtaccccaat gcaaagtggc 1500
   agcgtgtgaa gctacaggaa ggcaactctt gacaaaaccc cagcaccaat ttgttagacc 1560
   agatgtcaac tcttcttgtg gtgaagggta caagttaagt gggagtgttt atcaggagtg 1620
   tcaaggcaca attccttggt ttatggagat tcgtctttgt aaagaaatca cctgcccacc 1680
   accccctgtt atctacaatg gggcacacac cgggagttcc ttagaagatt ttccatatgg 1740
   aaccacggtc acttacacat gtaaccctgg gccagaaaga ggagtggaat tcagcctcat 1800
   tggagagagc accatccgtt gtacaagcaa tgatcaagaa agaggcacct ggagtggccc 1860
   tgctcccctg tgtaaacttt ccctccttgc tgtccagtgc tcacatgtcc atattgcaaa 1920
   tggatacaag atatctggca aggaagcccc atatttctac aatgacactg tgacattcaa 1980
   gtgttatagt ggatttactt tgaagggcag tagtcagatt cgttgcaaag ctgataacac 2040
   ctgggatcct gaaataccag tttgtgaaaa agaaacatgc cagcatgtga gacagagtct 2100
   tcaagaactt ccagctggtt cacgtgtgga gctagttaat acgtcctgcc aagatgggta 2160
   ccagttgact ggacatgctt atcagatgtg tcaagatgct gaaaatggaa tttggttcaa 2220
   aaagattcca ctttgtaaag ttattcactg tcaccctcca ccagtgattg tcaatgggaa 2280
   gcacacaggc atgatggcag aaaactttct atatggaaat gaagtctctt atgaatgtga 2340
   ccaaggattc tatctcctgg gagagaaaaa attgcagtgc agaagtgatt ctaaaggaca 2400
   tggatcttgg agcgggcctt ccccacagtg cttacgatct cctcctgtga ctcgctgccc 2460
   taatccagaa gtcaaacatg ggtacaagct caataaaaca cattctgcat attcccacaa 2520
   tgacatagtg tatgttgact gcaatcctgg cttcatcatg aatggtagtc gcgtgattag 2580
   gtgtcatact gataacacat gggtgccagg tgtgccaact tgtatcaaaa aagccttcat 2640
   agggtgtcca cctccgccta agacccctaa cgggaaccat actggtggaa acatagctcg 2700
   attttctcct ggaatgtcaa tcctgtacag ctgtgaccaa ggctacctgc tggtgggaga 2760
   ggcactcctt ctttgcacac atgagggaac ctggagccaa cctgcccctc attgtaaaga 2820
   ggtaaactgt agctcaccag cagatatgga tggaatccag aaagggctgg aaccaaggaa 2880
   aatgtatcag tatggagctg ttgtaactct ggagtgtgaa gatgggtata tgctggaagg 2940
   cagtccccag agccagtgcc aatcggatca ccaatggaac cctcccctgg cggtttgcag 3000
   atcccgttca cttgctcctg tcctttgtgg tattgctgca ggtttgatac ttcttacctt 3060
   cttgattgtc attaccttat acgtgatatc aaaacacaga gcacgcaatt attatacaga 3120
   tacaagccag aaagaagctt ttcatttaga agcacgagaa gtatattctg ttgatccata 3180
   caacccagcc agctgatcag aagacaaact ggtgtgtgcc tcattgcttg gaattcagcg 3240
   gaatattgat tagaaagaaa ctgctctaat atcagcaagt ctctttatat ggcctcaaga 3300
   tcaatgaaat gatgtcataa gcgatcactt cctatatgca cttattctca agaagaacat 3360
   ctttatggta aagatgggag cccagtttca ctgccatata ctcttcaagg actttctgaa 3420
   gcctcactta tgagatgcct gaagccaggc catggctata aacaattaca tggctctaaa 3480
   aagttttgcc ctttttaagg aaggcactaa aaagagctgt cctggtatct agacccatct 3540
   tctttttgaa atcagcatac tcaatgttac tatctgcttt tggttataat gtgtttttaa 3600
   ttatctaaag tatgaagcat tttctggggt tatgatggct ttacctttat taggaagtat 3660
   ggttttattt tgatagtagc ttcctcctct ggtggtgtta atcatttcat ttttaccctt 3720
   acttggtttg agtttctctc acattactgt atatactttg cctttccata atcactcagt 3780
   gattgcaatt tgcacaagtt tttttaaatt atgggaatca agatttaatc ctagagattt 3840
   ggtgtacaat tcaggctttg gatgtttctt tagcagtttt gtgataagtt ctagttgctt 3900
   gtaaaatttc acttaataat gtgtacatta gtcattcaat aaattgtaat tgtaaagaaa 3960
   acatacaaaa aaaaaaaaaa aa 3982

<210> 2
<211> 1531
<212> DNA
<213> Homo sapiens

<400> 2
agtcacagag ggaacacaga gcctagttgt aaacggacag agacgagagg ggcaagggag 60
gacagtggat gacagggaag acgagtgggg gcagagctgc tcaggaccat ggctgaggcc 120
atcacctatg cagatctgag gtttgtgaag gctcccctga agaagagcat ctccagccgg 180
ttaggacagg acccaggggc tgatgatgat ggggaaatca cctacgagaa tgttcaagtg 240
cccgcagtcc taggggtgcc ctcaagcttg gcttcttctg tactagggga caaagcagcg 300
gtcaagtcgg agcagccaac tgcgtcctgg agagccgtga cgtcaccagc tgtcgggcgg 360
attctcccct gccgcacaac ctgcctgcga tacctcctgc tcggcctgct cctcacctgc 420
ctgctgttag gagtgaccgc catctgcctg ggagtgcgct atctgcaggt gtctcagcag 480
ctccagcaga cgaacagggt tctggaagtc actaacagca gcctgaggca gcagctccgc 540
ctcaagataa cgcagctggg acagagtgca gaggatctgc aggggtccag gagagagctg 600
gcgcagagtc aggaagcact acaggtggaa cagagggctc atcaggcggc cgaagggcag 660
ctacaggcct gccaggcaga cagacagaag acgaaggaga ccttgcaaag tgaggagcaa 720
cagaggaggg ccttggagca gaagctgagc aacatggaga acagactgaa gcccttcttc 780

acatgcggct cagcagacac ctgctgtccg tcgggatgga taatgcatca gaaaagctgc 840
ttttacatct cacttacttc aaaaaattgg caggagagcc aaaaacaatg tgaaactctg 900
tcttccaagc tggccacatt cagtgaaatt tatccacaat cacactctta ctacttctta 960
aattcactgt tgccaaatgg tggttcaggg aattcatatt ggactggcct cagctctaac 1020
aaggattgga agttgactga tgatacacaa cgcactagga cttatgctca aagctcaaaa 1080
tgtaacaagg tacataaaac ttggtcatgg tggacactgg agtcagagtc atgtagaagt 1140
tctcttccct acatctgtga gatgacagct ttcaggtttc cagattagga cagtcctttg 1200
cactgagttg acactcatgc caacaagaac ctgtgcccct ccttcctaac ctgaggcctg 1260
gggttcctca gaccatctcc ttcattctgg gcagtgccag ccaccggctg acccacacct 1320
gacacttcca gccagtctgc tgcctgctcc ctcttcctga aactggactg ttcctgggaa 1380
aagggtgaag ccacctctag aagggacttt ggcctccccc caagaacttc ccatggtaga 1440
atggggtggg ggaggagggc gcacgggctg agcggatagg ggcggcccgg agccagccag 1500
gcagttttat tgaaatcttt ttaaataatt g 1531

<210> 3
<211> 1653
<212> DNA
<213> Homo sapiens

<400> 3
agcgatttca tcttcaggcc tggactacac cactcaccct cccagtgtgc ttgagaaaca 60
aactgcaccc actgaactcc gcagctagca tccaaatcag cccttgagat ttgaggcctt 120
ggagactcag gagttttgag agcaaaatga caacacccag aaattcagta aatgggactt 180
tcccggcaga gccaatgaaa ggccctattg ctatgcaatc tggtccaaaa ccactcttca 240
ggaggatgtc ttcactggtg ggccccacgc aaagcttctt catgagggaa tctaagactt 300
tgggggctgt ccagattatg aatgggctct tccacattgc cctggggggt cttctgatga 360
tcccagcagg gatctatgca cccatctgtg tgactgtgtg gtaccctctc tggggaggca 420
ttatgtatat tatttccgga tcactcctgg cagcaacgga gaaaaactcc aggaagtgtt 480
tggtcaaagg aaaaatgata atgaattcat tgagcctctt tgctgccatt tctggaatga 540
ttctttcaat catggacata cttaatatta aaatttccca ttttttaaaa atggagagtc 600
tgaattttat tagagctcac acaccatata ttaacatata caactgtgaa ccagctaatc 660
cctctgagaa aaactcccca tctacccaat actgttacag catacaatct ctgttcttgg 720
gcattttgtc agtgatgctg atctttgcct tcttccagga acttgtaata gctggcatcg 780
ttgagaatga atggaaaaga acgtgctcca gacccaaatc taacatagtt ctcctgtcag 840
cagaagaaaa aaaagaacag actattgaaa taaaagaaga agtggttggg ctaactgaaa 900
catcttccca accaaagaat gaagaagaca ttgaaattat tccaatccaa gaagaggaag 960

aagaagaaac agagacgaac tttccagaac ctccccaaga tcaggaatcc tcaccaatag 1020
aaaatgacag ctctccttaa gtgatttctt ctgttttctg tttccttttt taaacattag 1080
tgttcatagc ttccaagaga catgctgact ttcatttctt gaggtactct gcacatacgc 1140
accacatctc tatctggcct ttgcatggag tgaccatagc tccttctctc ttacattgaa 1200
tgtagagaat gtagccattg tagcagcttg tgttgtcacg cttcttcttt tgagcaactt 1260
tcttacactg aagaaaggca gaatgagtgc ttcagaatgt gatttcctac taacctgttc 1320
cttggatagg ctttttagta tagtattttt ttttgtcatt ttctccatca acaaccaggg 1380
agactgcacc tgatggaaaa gatatatgac tgcttcatga cattcctaaa ctatcttttt 1440
tttattccac atctacgttt ttggtggagt cccttttgca tcattgtttt aaggatgata 1500
aaaaaaaaat aacaactagg gacaatacag aacccattcc atttatcttt ctacagggct 1560
gacattgtgg cacattctta gagttaccac accccatgag ggaagctcta aatagccaac 1620
acccatctgt tttttgtaaa aacagcatag ctt 1653

<210> 4
<211> 1752
<212> DNA
<213> Homo sapiens

<400> 4
cgggggggcc cgaccaccgc ggggccggga cgcgatggcg gcggcagagc ccgcgagctc 60
gggccagcag gcgccggcag ggcaggggca gggccagcgg ccgccgccgc agcctccgca 120
ggcgcaagcc ccgcagccgc ccccgccgcc gcagctcggg ggcgcggggg gcggcagcag 180
caggcacgag aagagcctgg ggctgctcac taccaagttc gtgtcgctgc tgcaggaggc 240
caaggacggc gttctggatc tcaaagcggc tgctgatact ttggctgtga ggcaaaaaag 300
gagaatttat gatatcacca atgtcttaga gggaattgac ttgattgaaa aaaagtcaaa 360
aaacagtatc cagtggaaag gtgtaggtgc tggctgtaat actaaagaag tcatagatag 420
attaagatat cttaaagctg aaattgaaga tctagaactg aaggaaagag aacttgatca 480
gcagaagttg ttgctacagc aaagcatcaa aaatgtgatg gacgattcca ttaataatag 540
attttcctat gtaactcatg aagacatctg taattgcttt aatggtgata cacttttggc 600
cattcaggca ccttctggta cacaactgga ggtacccatt ccagaaatgg gtcagaatgg 660
acaaaagaaa taccagatca atctaaagag tcattcagga cctatccatg tgctgcttat 720
aaataaagag tcgagttcat ctaagcccgt ggtttttcct gttcccccac ctgatgacct 780
cacacagcct tcctcccagt ccttgactcc agtgactcca cagaaatcca gcatggcaac 840
tcaaaatctg cctgagcaac atgtctctga aagaagccag gctctgcagc agacatcagc 900
tacagatata tcttcagcag gatctattag tggagatatc attgatgagt taatgtcttc 960

tgacgtgttt cctctcttaa ggctttctcc taccccggca gatgactaca actttaattt 1020
agatgataac gaaggagttt gtgatctgtt tgatgtccag atactaaatt attagattcc 1080
atggaaactt gggactgtta tctacctcta actgtgtaac atttagactt tcttaataac 1140
ctaaatattt aaaataatga atgtaacacc ttttttagtt cactgattct gaagtgttct 1200
tccctaatac tttctttact tcacaaaact tcaaccataa aaacaaaggg ctctgattgc 1260
tttaggggat aagtgattta atattcacaa acgtccccac tcccaaaagt aactatattc 1320
tggatttcaa cttttcttct aattgtgaat ccttccgttt tttcttctta aggaggaaag 1380
ttaaaggaca ctacaggtca tcaaaaacaa gttggccaag gactcattac ttgtcttata 1440
tttttactgc cactaaactg cctgtatttc tgtatgtcct tctatccaaa cagacgttca 1500
ctgccacttg taaagtgaag gatgtaaacg aggatatata actgtttcag tgaacagatt 1560
ttgtgaagtg ccttctgttt tagcacttta agtttatcac attttgttga cttctgacat 1620
tccactttcc taggttatag gaaagatctg tttatgtagt ttgtttttaa aatgtgccaa 1680
tgcctgtaca ttaacagatt tttaaaaata aaattgtata aaacattaaa aaaaaaaaaa 1740
aaaaaaaaaa aa 1752

## Description

This invention relates to a method of normalizing gene expression data, wherein said gene expression data are obtained from a sample out of a plurality of samples, the sample reflecting one or more cell type(s), and the method comprising the steps of: (a) determining the expression level in said sample of one or more first genes the expression of which is specific for one or more chosen cell type(s) and is constant per cell across said plurality of samples (constant marker genes); and (b) normalizing the expression level of one or more second genes in said sample using the expression level(s) determined in step (a). Also provided is a computer program comprising program code means for performing the method of the invention.

In this specification, a number of documents is cited. The disclosure of these documents, including manufacturer's manuals, is herewith incorporated by reference in its entirety.

Several techniques for analyzing alterations in gene expression profiles have been developed over the past 20 years, including Northern blot, serial analysis of gene expression (SAGE), differential display PCR (DD-PCR) and representational difference analysis (RDA). Nowadays, microarrays or DNA-chips provide a more sophisticated technology to measure differential gene expression which allows high throughput and the identification of marker genes in clinical samples. The markers then can be introduced to medical diagnosis and therapy. By applying microarray experiments, the amount of transcripts can be measured for all those transcripts where complementary probes are immobilized on the array. Up to several thousands of genes can by analyzed in parallel on microarrays. Typically, microarray experiments involve a comparison of the amount of mRNA in a sample to be analyzed (e.g. a sample of tumor tissue) and in a control or reference sample (e.g. normal/healthy tissue).

Unfortunately, the occurrence of homogeneous samples is rare. In most clinical applications the samples that are analyzed, e.g. tumor tissue, biopsies or blood samples, contain a mixture of different cell types. Little is know about the distribution of the different cell types (tumor cells, epithelial cells or different immune cells) in such a sample. However, in order to correctly interpret an experiment, it is important to know whether an observed change in gene expression is due to differences in the relative amount of different cell types present in the samples compared, or due to a biological process occurring in one or more cell types, possibly indicative of a disease. Even if the expression of a particular gene can be assigned to a specific cell type, two scenarios can be envisaged which both may account for differential gene expression, viz. (i) the amount of that specific cell type is identical in both samples and the genes are differentially expressed in these cells, or (ii) the number of specific cell types is different in the samples and the gene expression is not truly altered. In scenario (i) there might be an important role for genes that are differentially expressed, e.g. they might be causative of tumor growth, whereas in situation (ii) differences in the measured gene expression are the result of the different distributions of cell types in the samples compared.

Flow cytometry and other wet-lab techniques, such as histological analysis, ELlspot and in situ PCR, for quantifying cell types in blood samples or tissues are well established. They allow analyses of proportions of cell populations as well as quantification of biological markers, e.g. the protein expression thereof. Being highly specific and sensitive, they permit the detection of small amounts of protein expressing cells, even at the single cell level. Fluorescence-activated cell sorting (FACS) is being applied for either characterizing a cell population or for generating almost homogeneous cell populations. However, current technologies are limited by the need for specific antibodies which are not available for the majority of gene products.
Laser micro-dissection (LMD) is another technique for either characterizing a cell population or generating almost homogeneous cell populations. Laser micro-dissection is a very reliable technique but generates only very limited amounts of starting material. Therefore, generally the amplification of mRNA from those samples is necessary. Amplification in turn has the disadvantage that precise linear amplification is difficult to accomplish. As a consequence, differential expression as observed for samples undergone amplification may not necessarily provide a faithful picture of differential expression as it applies to the corresponding not amplified sample, expression data for which would not be available though.

US patent application US2003/0215807 describes a method of normalizing two or more molecular array data sets. In a first step, the data sets are globally normalized, for example by dividing signal magnitudes by the chip-averaged signal magnitude. Subsequently, signal magnitudes are ranked and, using only signal magnitudes of those features on the array where the rank is similar across the data sets, a recalibration of the signal magnitudes is performed. While intending to provide a more robust estimate of the mean intensity, the notion of sample heterogeneity is absent from US2003/0215807 as is any attempt to overcome the problems arising from said sample heterogeneity.

The international patent application W02004/000867 describes a method for assessing, monitoring and/or determining the phenotype of cells and tissues using microarrays. So-called phenotype-specific genes (PSG) are introduced for the purpose of providing focused microarrays (see e.g. Example 3). As regards normalization, general normalization (in the art also referred to as global normalization, see also first step of the procedure described in US2003/0215807) and selected normalization are discussed. The latter makes use of a subset of genes, viz. housekeeping genes (HSKs) which are considered to be constantly expressed across all cell types. However, the application fails to address or even raise the issue of sample heterogeneity.

ln view of the limitations of the methods described in the prior art, the technical problem underlying the present invention was therefore the provision of means and methods for the analysis of gene expression in heterogeneous samples.

Accordingly, this invention relates to a method of normalizing gene expression data, wherein said gene expression data are obtained from a sample out of a plurality of samples, the sample reflecting one or more cell type(s), and the method comprising the steps of: (a) determining the expression level in said sample of one or more first genes the expression of which is specific for one or more chosen cell type(s) and is constant per cell across said plurality of samples (constant marker genes); and (b) normalizing the expression level of one or more second genes in said sample using the expression level(s) determined in step (a).

The term "normalizing" in relation to expression data is common in the art and relates to a processing step of the raw expression data which renders the signal intensities of each gene comparable across multiple measurements. Expression levels of a particular gene may differ between samples for a variety of reasons. Reasons of particular relevance are different amounts of cells and/or RNA in the samples analyzed on the one side and different transcriptional activity of the gene(s) under consideration on the other side. While the former is generally not indicative of a distinct biological state of the samples being compared, the latter generally is. In case protein expression levels are monitored instead of or in addition to mRNA expression levels, different transcriptional and/or translational activity may contribute to different protein expression levels. Meaningful analysis of expression data requires the two possible contributions to changes in expression levels - amount of cells and/or RNA vs. transcriptional and/or translational activity - to be disentangled. Normalization is a method for disentangling said contributions. Practically speaking, normalization is a transformation of the raw expression data such that the effect of different amounts of cells and/or of RNA is removed or substantially removed. Global normalization, a procedure well known in the art, for example involves (i) the determination of the average signal intensity across all genes whose expression is being measured and (ii) subsequent division of raw signal intensities by the average signal intensity obtained in step (i).
Further reasons necessitating normalization include differences in labeling or detection efficiencies between the fluorescent dyes used. In experiments where two fluorescent dyes (for example red and green, i.e., Cy5 and Cy3) have been used, intensity-dependent variation in dye bias may introduce spurious variations in the collected data. Lowess normalization applies a smoothing adjustment that removes such variation.

The term "expression data" refers to quantitative data characterizing the mRNA expression level and/or the protein expression level of one or more genes.

Preferably, said expression data are proportional to the number of mRNA or protein molecules, respectively, present in the analysed sample. mRNA expression levels and/or differences between the mRNA expression levels of a particular gene in distinct samples may be obtained by a variety of methods known in the art including PCR, RT-PCR and experiments using microarrays or DNA chips which are further detailed below, and furthermore Northern blotting, serial analysis of gene expression (SAGE), differential display PCR (DD-PCR) and representational difference analysis (RDA).
The skilled person is aware of methods for the quantitation of proteins. Expression levels of purified protein in solution can be determined by physical methods, e.g. photometry. Methods of determining the expression level of a particular protein in a mixture rely on specific binding, e.g of antibodies. Specific detection and quantitation methods exploiting the specificity of antibodies comprise immunohistochemistry (*in situ)* and surface plasmon resonance. Western blotting combines separation of a mixture of proteins by electrophoresis and specific detection with antibodies. Other means of determining protein expression data include two-dimensional gel-electrophoresis, preferably in combination with mass spectrometry. With the advent of microarray technology, measurement of protein expression levels in array format became increasingly widespread. Protein arrays for determining protein expression data exploit interactions such as protein-antibody, protein-protein, protein-ligand, protein-drug and protein-small molecule interactions or any combination thereof. Protein expression data reflect, in addition to regulation at the transcriptional level, regulation at the translational level as well as the average lifetime of a protein prior to degradation.

The term "signal intensity" as used herein refers to a measured quantity indicative of the expression level of a gene. Preferably, the signal intensity is proportional to the amount of transcript or the amount of protein translated from a gene. Depending on the label used, which is further detailed below, the signal may be light emitted by a fluorescence or luminescent process, or radiation and/or particles emitted by a radioactive label or dye (quantum dots).

The term "raw expression data" specifically refers to expression data prior to normalization. For example, and in the case of expression data obtained from microarrays, raw expression data are the data obtained from the image processing of the scanned hybridized microarray.

The term "multiple measurements" refers to multiple determinations of an expression level of a given gene, typically for multiple samples. Each sample may be hybridized to a different chip, as it is the case for oligonucleotide chips such as those available from Affymetrix. Alternatively, two samples to be compared may be labelled differently, for example by using a different fluorescent dye for each sample, and both samples may be hybridized onto the same chip. The latter is typically the case for cDNA microarrays or oligonucleotide chips.

The term "sample" refers to an aliquot taken from a biological system. A sample according to the invention may undergo several processing steps. Particularly envisaged is the isolation of RNA, optionally the separation of polyadenylated RNA (polyA+ RNA), furthermore the transcription of RNA into cDNA, and, if necessary, the amplification of the cDNA. If protein expression levels are to be monitored, said processing comprises the isolation of protein. A sample according to the invention reflecting more than one cell type is herein also referred to as a heterogeneous sample. It comprises or is derived from a plurality of cell types. Alternatively, samples may be derived from or contain one cell type.

Said first genes, also referred to as constant marker genes, permit the determination of a quantity proportional to the number of cells of said one or more chosen cell type(s) present in a sample. This proportionality results (i) from the expression level of said first genes being constant per cell across said plurality of samples and (ii) from the expression being specific for said one or more chosen cell type(s). The fact that said first genes are constantly and cell type-specifically expressed is prior knowledge for the purpose of this invention. It is sufficient that said genes are constantly expressed across the set of samples under consideration. Nevertheless, it is envisaged that said first genes are constantly expressed in said one or more chosen cell type(s) under most or all conceivable conditions. The term "constant per cell" means that each cell of said one or more chosen cell type(s) expresses the same or substantially the same amount of transcript and/or protein from said first genes. The term "specific for one or more chosen cell type(s)" in relation to expression designates genes whose detectable expression is confined or substantially confined to said one or more chosen cell type(s).

The term "chosen cell type(s)" may refer to a subset of the cell types present in the sample. Alternatively, the chosen cell types may embrace all cell types present in the sample. In both cases, the chosen cell type(s) is/are (a) cell types for which constant marker genes are known. In case of a plurality of chosen cell types, these constant marker genes are constantly expressed in all chosen cell types, preferably at identical or substantially identical levels across the different cell types comprised in the set of chosen cell types.

The term "second gene" according to the invention refers to any gene the expression level of which has been determined and shall be subjected to the method of normalizing according to the invention. The term comprises genes which may be differentially regulated across said plurality of samples.

Step (a) of the method of normalizing according to the invention relates to the determination of a quantity which is proportional to the number of cells of a specific cell type or of a plurality of cell types, wherein said cell type(s) may be a subset of all cell types present or reflected in the sample. Alternatively, said cell type(s) are all cell types present or reflected in the sample.
In both cases, step (a) provides a means of counting cells which advantageously does not require performing further experiments, such as flow cytometry. This is particularly advantageous in those cases where only small amounts of samples are available, or where samples are not available at all, for example after completion of a study or consumption of all sample material. The method of the invention furthermore permits the re-analysis of expression data sets from previously completed studies.
Once the number of cells (or, more precisely, a quantity proportional thereto) is determined, it may be used for normalizing. The result of the normalization is an expression level per cell (or, more precisely, a quantity proportional thereto). It is particularly envisaged that said normalization is applied to those second genes whose expression, as that of said first genes, is specific or substantially specific for said one or more chosen cell type(s), while the expression of said second genes, as opposed to said first genes, may vary or be constant across the measured samples.

As regards step (b), normalization as such is a step of processing expression data well known in the art. Preferred ways of performing the normalization of step (b) are further detailed below.

Surprisingly, the method of normalizing according to the invention may lead to the identification of differentially regulated genes which, upon normalizing using state-of-the-art global normalization, would not have been identified as being differentially regulated. Furthermore, genes which were believed to be differentially expressed based on an analysis of expression data which have been normalized by normalization methods known in the art such as global normalization, may turn out not to be truly differentially expressed when normalizing the data set using the method of the invention, thereby accounting for sample heterogeneity and/or different amounts of cells in the samples analyzed. In other words, the method of the invention leads to less false positive and less false negative determinations of differential expression than commonly used normalization methods. This surprising finding is of particular relevance for the analysis of clinical samples, noting (i) that clinical samples, as described herein above, frequently are heterogeneous and (ii) that the identification of differentially regulated genes is of paramount importance, given the need for targets for specific therapeutic intervention and for diagnostic markers.

In a preferred embodiment, the sample reflects more than one cell type.

In a further preferred embodiment, the chosen cell type(s) is/are a subset of the cell types in said sample. In this embodiment, the method of the invention comprises the quantitative determination of a sub-population of cells present in the sample.

In a further preferred embodiment, the method of the invention comprises the further step(s) of: repeating steps (a) and (b), (c) for one or more further cell type(s) or one or more further subset(s) of cell types; and/or (d) for one or more further samples.
This embodiment comprises dissecting heterogeneous samples into discrete subpopulations. In other words, the method of the invention permits to define the composition of samples with regard to cell types.

In a further preferred embodiment, said expression data are mRNA expression data or protein expression data.

In a more preferred embodiment, said mRNA expression data are obtained (e) by hybridizing said sample on a microarray or DNA chip exhibiting probes complementary to at least part of the nucleic acids comprised in said sample; and/or (f) by performing quantitative real-time PCR.

The term "chip" as used herein embraces a miniaturized device carrying a plurality of distinct biological species such as biological molecules attached to distinct sites of its surface. The arrangement of said sites may be regular, such arrangement being also referred to as an array. Accordingly, this type of chip is also referred to as array or microarray. For the purpose of measuring mRNA expression data, DNA chips such as those manufactured by Affymetrix carrying oligonucleotides on their surface as well as microarrays carrying cDNAs obtained from any library are commonly used in the art. Furthermore, said chips or microarrays may also carry PNA molecules as probes.
The term "PNA" stands for "peptide nucleic acid". In brief, a PNA is a synthetic DNA-mimic with an amide backbone in place of the sugar-phosphate backbone of DNA or
RNA (Nielsen et al. (1991) Science, 254, 1497-1500; Egholm et al. (1993), Nature, 365, 566-568). PNAs exhibit several advantageous features. They are stable under acidic conditions and resistant to nucleases as well as proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060). Thus, PNA oligomers can be shorter than oligonucleotides when used as hybridisation probes. On the other hand, mismatches have a more destabilising effect, thus improving discrimination between perfect matches and mismatches. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to probe molecules. Polynucleotides to be hybridized to probes on a DNA chip or microarray typically carry a label such as a fluorescent label, thereby allowing for subsequent detection and quantification of hybridized material.
Specific fluorescent labels according to the invention include the following commercially available (for example from Synthegen) fluorescent dyes: Tamra-dT, 5-Fluorescein (FITC), 5-Carboxyfluorescein (FAM), 6-Carboxyfluorescein (FAM), 3' 6-Carboxyfluorescein (FAM), 6-Carboxyfluorescein-DMT (FAM-X), 5(6)-Carboxyfluorescein (FAM), 6-Hexachlorofluorescein (HEX), 6-Tetrachlorofluorescein (TET), JOE, LightCycler Red 640, LightCycler Red 705, FAR-Fuchsia (5'-Amidite), FAR-Fuchsia (SE), FAR-Blue (5'-Amidite), FAR-Blue (SE), FAR-Green One (SE), FAR-Green Two (SE), Oregon Green 488, Oregon Green 500, Oregon Green 514, BODIPY FL-X, BODIPY FL, BODIPY-TMR-X, BODIPY R6G, BODIPY 650/665, BODIPY 564/570, BODIPY 581/591, BODIPY TR-X, BODIPY 630/650, BODIPY 493/503, Carboxyrhodamine 6G, MAX, 5(6)-Carboxytetramethylrhodamine (TAMRA), 6-Carboxytetramethylrhodamine (TAMRA), 5(6)-Carboxy-X, Rhodamine (ROX), 6-Carboxy-X-Rhodamine (ROX), AMCA-X (Coumarin), Texas Red-X, Rhodamine Red-X, Marina Blue, Pacific Blue, Rhodamine Green-X, 7-diethylaminocoumarin-3-carboxylic acid, 7-methoxycoumarin-3-carboxylic acid, Cy3, Cy3B, Cy5, Cy5.5, DY-505, DY-550, DY-555, DY-610, DY-630, DY-633, DY-636, DY-650, DY-675, DY-676, DY-681, DY-700, DY-701, DY-730, DY-750, DY-751, DY-782, Cy3.5 and EDANS.
Alternatively, said label may be a luminescent or radioactive label or quantum dots.
The hybridized arrays are scanned and the obtained images are processed using image processing methods known in the art and available from a variety of suppliers in order to yield raw expression data. The method of the invention may also comprise the step of image processing.

PCR is well known in the art and is employed to make large numbers of copies of a target sequence. PCR comprises three major steps, which typically are repeated for 30 or 40 cycles. This is done on an automated cycler device, which can heat and cool containers with the reaction mixture in a very short time. The first step is denaturation, e.g. at 94°C. During the denaturation, the double strand melts to form single stranded DNA. Concomitantly, all enzymatic reactions are stopped. These enzymatic reactions include, for example, the extension reaction of a previous PCR cycle. The second step is annealing, e.g. at 54°C. Bonds are constantly formed and broken between the single stranded primer and the single stranded template. The more stable bonds last a bit longer (primers that match exactly) and on the formed double stranded DNA comprising template and primer, the polymerase can attach and starts copying the template. Once a few bases are built in, the bonds are sufficiently strong between the template and the primer such that it does not break anymore. The third step is an extension step which is performed at a temperature of, for example, 72°C. This is the ideal temperature for polymerase activity. The primers, where there are a few bases built in, already exhibit a stronger binding to the template as compared to primers that are on positions with no exact match, which in turn get loose again (because of the higher temperature as compared to the annealing step) and do not give rise to an extension of the fragment.
Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor which was formerly quenched due to the close proximity to the 3' acceptor in the TaqMan probe sequence. Thereby, the process of amplification can be monitored directly and in real time, which permits a significantly more precise determination of expression levels than conventional end-point PCR.

The term "hybridizing" as used herein refers to a pairing of oligo- or polynucleotides to a complementary strand of polynucleotide which thereby form a hybrid. Said oligo- or polynucleotides may be useful as probes in Northern or Southern Blot analysis of RNA or DNA preparations, respectively, or can be used as primers in PCR analysis or probes on a mircorarray dependent on their respective size. Preferably, said hybridizing polynucleotides comprise at least 10, more preferably at least 15, 20, 25, 30, 40 or 50 nucleotides in length.
It is well known in the art how to perform hybridization experiments with nucleic acid molecules, i.e. the person skilled in the art knows what hybridization conditions s/he has to use in accordance with the present invention. Such hybridization conditions are referred to in standard text books such as "Molecular Cloning A Laboratory Manual", Cold Spring Harbor Laboratory (1989) N.Y. or Higgins, S.J., Hames, D. "RNA Processing: A practical approach", Oxford University Press (1994), Vol. 1 and 2.

"Stringent hybridization conditions" refers to conditions which comprise, e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20µ/ml denatured, sheared salmon sperm DNA, followed by washing the filters in 0.1 x SSC at about 65°C. Said conditions for hybridization are also known by a person skilled in the art as "high stringent conditions for hybridization". Also contemplated are nucleic acid molecules that hybridize to the polynucleotides of the invention at lower stringency hybridization conditions ("low stringent conditions for hybridization"). Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1X SSPE, 0.1% SDS. In addition, to achieve even lower stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. '

In a preferred embodiment, the samples form two or more sets of samples. In a more preferred embodiment, one set of samples consists of one or more control or reference samples and another set of samples consists of one or more test samples. In a yet more preferred embodiment, said control or reference samples are obtained from healthy individuals and said test samples are obtained from diseased individuals. In a yet more preferred embodiment, said control or reference samples are obtained from diseased individuals suffering from a first disease or a first subtype of a disease and said test samples are obtained from diseased individuals suffering from a second disease or a second subtype of a disease.

In a further more preferred embodiment, said samples are samples obtained from tumours, epithelial cells, cells of the immune system, inflamed tissue, or blood. In a further more preferred embodiment, the chosen cell type is selected from the group consisting of tumour cells, epithelial cells, cells of the immune system or peripheral blood mononuclear cells such as B lymphocytes.

In a further preferred embodiment, normalization is effected by division with a factor, wherein said factor is specific for each sample.

In a more preferred embodiment, said factor is the expression level of a first gene as defined in (a). This embodiment of the method of the invention relates in particular to cases where only a single constant marker gene is known or has been measured.

In a further more preferred embodiment, said factor is the average of expression levels of a plurality of first genes as defined in (a), whereby the average is determined across said first genes. The term "average" embraces all specific definitions thereof known in the art. In particular, it embraces arithmetic mean and geometric mean of said expression levels of a plurality of first genes. Furthermore, the term comprises weighted averages, i.e., the individual expression levels contributing to the average are multiplied with different weighting factors, thereby allowing to account for, as an example, different confidence in the expression levels of different genes.

In a further more preferred embodiment, said expression level(s) is/are averaged across more than one sample. Averaging data obtained from a plurality of samples is a means of obtaining statistically more significant data. Preferably, also the expression levels of said one or more second genes are averaged across a plurality of samples, more preferred across the same set of samples used to derive average expression levels of said one or more first genes.

In a further preferred embodiment, normalization is effected by using a scatterplot smoothing algorithm, more preferred by using the locally weighted scatterplot smoothing (LOWESS) algorithm (Cleveland, 1979; Cleveland, 1981). Lowess regression, or locally weighted least squares regression, is a technique for fitting a smoothing curve to a dataset. The degree of smoothing is determined by the window width parameter. A larger window width results in a smoother curve, a smaller window results in more local variation.

In a further preferred embodiment, the method of the invention further comprises (g) determining the amount of cells of said chosen cell type(s), thereby providing an external control. Although it is distinct advantage of the method of the present invention that no further wet-lab experimentation is required for the determination of the amount of cells, it is nevertheless envisaged to determine the amount of cells by experimental methods for the purpose of validating the method and/or providing an external reference value.

In a more preferred embodiment, said determining the amount of cells is effected by a method for cell and/or tissue phenotyping such as flow cytometry, fluorescence in-situ hybridization, laser capture micro-dissection, histology or in situ PCR.

In a further preferred embodiment, said first gene(s) is/are selected from the group consisting of genes encoding mRNA sequences as set forth in SEQ ID NO: 1 to 4. Said sequences correspond to GenBank databases entries NM.001877, NM.001782, NM.021950 and NM.001951.

In a preferred embodiment, the method of the invention comprises the further step of (h) determining those second gene(s) the expression level(s) of which differ(s) between said sets of samples, wherein the difference occurs between one or more first set(s) of samples on the one side and the remaining set(s) of samples on the other side; thereby identifying (a) marker gene(s) for said first set(s) of samples.
It is understood that the markers identified by using this embodiment of the invention are to be distinguished from the constant marker genes used by the method in the first step. While the constant marker genes are known a priori and expressed at a constant level per cell in all samples, said marker gene(s) for said first set(s) of samples are identified as an outcome of the method according to this embodiment and are not expressed at a constant level per cell across all samples.

In a more preferred embodiment, at least one set of samples consists of (a) sample(s) obtained from (a) diseased individual(s), and wherein in step (h) (a) disease marker gene(s) is/are identified. Disease marker gene(s) may either be up- or down-regulated in the group of diseased individual(s) as compared to healthy individuals.
As stated herein above, the identification of markers, and, notabene the accurate identification thereof, is urgently needed, both for diagnostic applications as well as for therapeutic approaches in those case where the markers lend themselves as targets for therapeutic interference.

In a yet more preferred embodiment, the method comprises the further steps of (i) determining whether any known disease marker gene(s) is/are present among the disease marker gene(s) identified by the above method; and, if present, (j) diagnosing the disease.
Here it is envisaged to relate the disease markers determined using the method of the invention for a given expression data set to previously established disease markers. If one or more of said previously established disease markers are identified among the disease markers determined using the method of the invention, the method proceeds to diagnose the disease.

The present invention also provides a computer program comprising program code means for performing the method of the invention when the program is run on a computer.
Also provided is a computer readable storage medium for storing a computer program product for performing the method of the invention.

The Figures show:

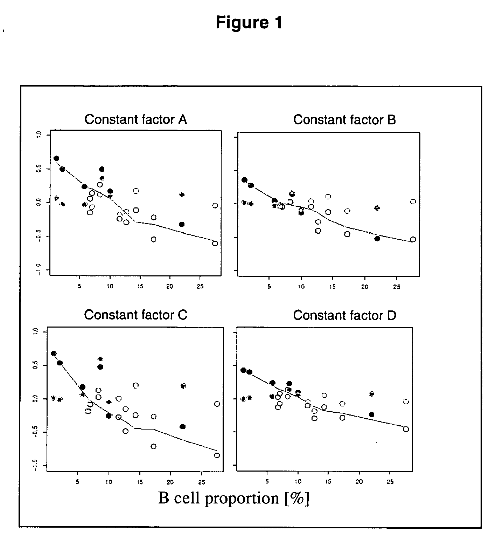

**Figure 1:** Comparison of B cell-specific constant markers A, B, C, and D with the proportion of B cells within a PBMC population (see also Example 1).

The following examples illustrate the invention but should not be construed as being limiting.

### Example 1

The influence of differences in cell proportions on microarray-based gene expression profiling is shown for B lymphocyte constant markers in Figure 1. Differences of gene expression for individual donors compared to a common reference RNA pool of four B cell specific constant markers (A, B, C, and D) are shown on the y-axis (log 10 ratio (comparison to reference)). B cell proportions within peripheral blood mononuclear cells (PBMC) determined by flow cytometry (FACS) are shown on the x-axis.
Results of gene expression data obtained from six tuberculosis patients (filled blue circles) and eight healthy individuals (open blue circles) using global gene expression analysis suggest differences in the expression of B cell-specific constant markers between both study groups. Analyses of B cell proportion and microarray data in individual donors revealed significant correlation of B cell-specific constant markers A, B, C, and D with the B cell percentage. This correlation is apparent from the continuous graphs (red lines) shown in Figure 1 which are the result of the Lowess scatter-plot smoothing algorithm (program code in the script language R is provided below) applied to the data set consisting of expression data resulting from global gene expression analysis and corresponding B cell proportions. Here, the Lowess method is used for de-confounding the data set and identifying significant trends in the data.
However, after normalizing the gene expression data to B cell proportion as reflected by markers A, B, C and D using the method of the invention, no significant expression difference was detected any more according to the Wilcoxon rank sum test for significant difference (patients, filled green circles; healthy individuals, open green circles).
In other words, false positive results obtained upon global normalization are rectified upon using the method of normalizing of the present invention. Rather, the normalization method of the invention excludes genuine gene expression differences between both study groups and furthermore demonstrates a correlation between the expression levels of B cell-specific constant markers and the B cell proportion in a sample. Therefore markers A, B, C, and D, assumed to be B cell-specific constant markers at the outset of the study, are confirmed as constant marker genes for this cell type. These results clearly indicate that the alteration in gene expression due to a disease can be monitored much more precise and false positive results can be avoided if the expression of constantly expressed cell type-specific factors are taken into consideration.

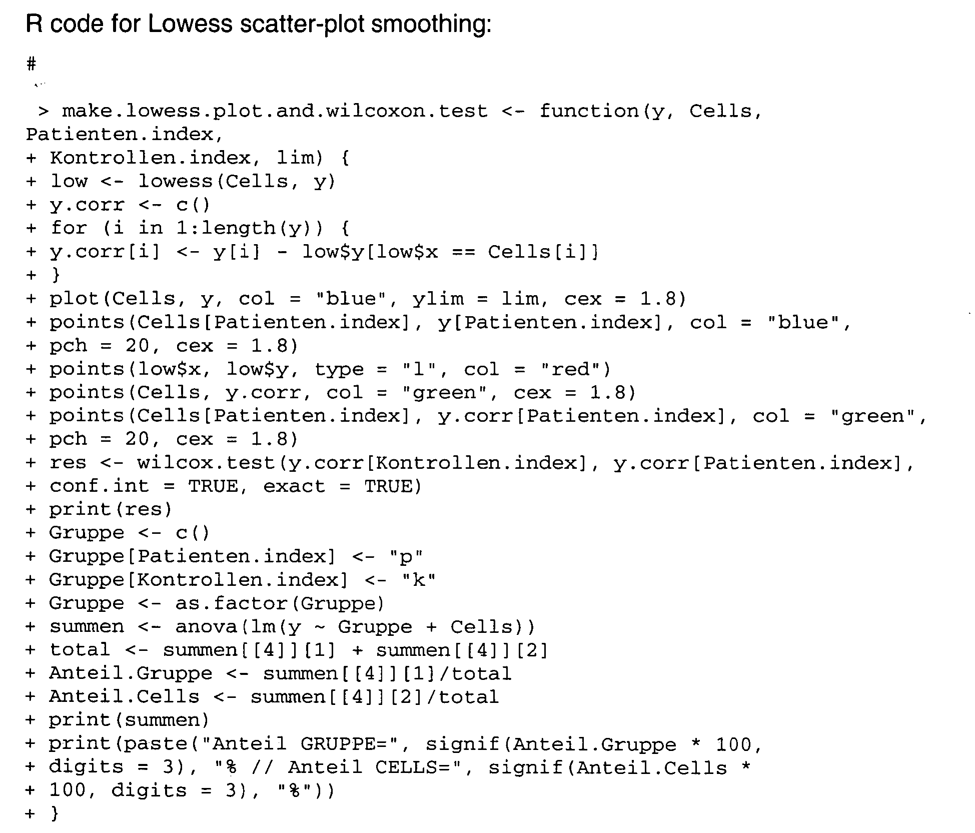

### References

Cleveland, W. S. (1979) Robust locally weighted regression and smoothing scatterplots. J. Amer. Statist. Assoc. 74, 829-836.

Cleveland, W. S. (1981) LOWESS: A program for smoothing scatterplots by robust locally weighted regression. The American Statistician, 35, 54.

## Claims

1. A method of normalizing gene expression data, wherein said gene expression data are obtained from a sample out of a plurality of samples, the sample reflecting one or more cell type(s), and the method comprising the steps of:
(a) determining the expression level in said sample of one or more first genes the expression of which is specific for one or more chosen cell type(s) and is constant per cell across said plurality of samples (constant marker genes); and
(b) normalizing the expression level of one or more second genes in said sample using the expression level(s) determined in step (a).

2. The method of claim 1, wherein the sample reflects more than one cell type.

3. The method of claim 2, wherein said chosen cell type(s) is/are a subset of the cell types in said sample.

4. The method of any one of claims 1 to 3, comprising the further step(s) of:
repeating steps (a) and (b)
(c) for one or more further cell type(s) or one or more further subset(s) of cell types; and/or
(d) for one or more further samples.

5. The method of any one of claims 1 to 4, wherein said expression data are mRNA expression data or protein expression data.

6. The method of claim 5, wherein said mRNA expression data are obtained
(e) by hybridizing said sample on a microarray or DNA chip exhibiting probes complementary to at least part of the nucleic acids comprised in said sample; and/or
(f) by performing quantitative real-time PCR.

7. The method of any one of claims 1 to 6, wherein the samples form two or more sets of samples.

8. The method of claim 7, wherein one set of samples consists of one or more control or reference samples and another set of samples consists of one or more test samples.

9. The method of claim 8, wherein said control or reference samples are obtained from healthy individuals and said test samples are obtained from diseased individuals.

10. The method of claim 8, wherein said control or reference samples are obtained from diseased individuals suffering from a first disease or a first subtype of a disease and said test samples are obtained from diseased individuals suffering from a second disease or a second subtype of a disease.

11. The method of any one of claims 1 to 10, wherein said samples are samples obtained from tumours, epithelial cells, cells of the immune system, inflamed tissue, or blood.

12. The method of any one of claims 1 to 11, wherein the chosen cell type is selected from the group consisting of tumour cells, epithelial cells, cells of the immune system or peripheral blood mononuclear cells such as B lymphocytes.

13. The method of any one of claims 1 to 12, wherein normalization is effected by division with a factor, wherein said factor is specific for each sample.

14. The method of claim 13, wherein said factor is the expression level of a first gene as defined in (a).

15. The method of claim 13, wherein said factor is the average of expression levels of a plurality of first genes as defined in (a), whereby the average is determined across said first genes.

16. The method of claim 14 or 15, wherein said expression level(s) is/are averaged across more than one sample.

17. The method of any one of claims 1 to 12, wherein normalization is effected by using the locally weighted scatterplot smoothing (LOWESS) algorithm.

18. The method of any one of claims 1 to 17, further comprising
(g) determining the amount of cells of said chosen cell type(s), thereby providing an external control.

19. The method of claim 18, wherein said determining the amount of cells is effected by a method for cell and/or tissue phenotyping such as flow cytometry, fluorescence in-situ hybridization, laser capture micro-dissection, histology or in situ PCR.

20. The method of any one of claims 1 to 19, wherein said first gene(s) is/are ' selected from the group consisting of genes encoding mRNA sequences as set forth in SEQ ID NO: 1 to 4.

21. The method of any one of claim 7 to 20, comprising the further step of
(h) determining those second gene(s) the expression level(s) of which differ(s) between said sets of samples, wherein the difference occurs between one or more first set(s) of samples on the one side and the remaining set(s) of samples on the other side;
thereby identifying (a) marker gene(s) for said first set(s) of samples.

22. The method of claim 21, wherein at least one set of samples consists of (a) sample(s) obtained from (a) diseased individual(s), and wherein in step (h) (a) disease marker gene(s) is/are identified.

23. The method of claim 22, comprising the further steps of
(i) determining whether any known disease marker gene(s) is/are present among the disease marker gene(s) identified by the method of claim 21; and, if present,
(j) diagnosing the disease.

24. A computer program comprising program code means for performing the method of any of the preceding claims when the program is run on a computer.
